# EUROPÄISCHE PATENTANMELDUNG

(11) **EP 3 245 943 A1**
(43) Veröffentlichungstag der Anmeldung: **22.11.2017**
(21) Anmeldenummer: 16199774.7
(22) Anmeldetag: 21.11.2016
(51) Int. Cl.: A61B 5/00, A61B 5/08, A61B 5/107, A61B 5/11, A61B 5/113

(54) **VERFAHREN ZUR BERÜHRUNGSLOSEN ERMITTLUNG UND AUFBEREITUNG VON SCHLAFBEWEGUNGSDATEN**

(30) Priorität: 18.05.2016 EP 16170190
(71) Anmelder: Motognosis UG (haftungsbeschränkt), 10119 Berlin (DE)
(72) Erfinder: KAYSER, Bastian, 10439 Berlin (DE); OTTE, Karen, 10365 Berlin (DE); MANSOW-MODEL, Sebastian, 12683 Berlin (DE); BRANDT, Alexander, 46487 Wesel (DE)
(74) Vertreter: Schulz Junghans Patentanwälte PartGmbB

(57) **Zusammenfassung**

Die Erfindung betrifft unter anderem ein Verfahren zur berührungslosen Ermittlung von Atem- und Beinbewegungen schlafender Personen mit einem Tiefensensor, umfassend die Schritte:
- Bereitstellen einer insbesondere vorverarbeiteten Zeitserie von Tiefenbildern des Tiefensensors, die einen Schlafplatz mit der schlafenden Person umfassen,
- Ermitteln und Quantifizieren zumindest eines Bewegungssignals, das aus Bewegungen mindestens eines Körperbereichs der schlafenden Person aus der Zeitserie der Tiefenbilder ermittelt wird, wobei der mindestens eine Körperbereich den Torso, insbesondere den Brustbereich der Person umfasst und/ oder wobei der mindestens eine Körperbereich den Beinbereich der Person umfasst.

## Beschreibung

Die Erfindung betrifft ein Verfahren zur berührungslosen Ermittlung von Atem- und Beinbewegungen, zur berührungslosen Klassifikation von Schlafphasen und Schlafpositionen einer Person mit einem Tiefensensor, sowie ein Verfahren zur Analyse der so ermittelten Schlafparameter.

Schlaf ist von größter Wichtigkeit für die physische und psychische Gesundheit des Menschen. Daher ist die Untersuchung und Überwachung von Schlaf von besonderer Wichtigkeit für die Diagnose und Behandlung von Schlafkrankheiten.

Klassische Methoden der Schlafüberwachung, d.h. die Polysomnographie, sind sehr teuer, aufwendig und beeinträchtigen das normale Schlafverhalten von Patienten, da diese hierfür umfangreich verkabelt und mit Elektroden beklebt werden müssen.

Die Aufgabe der Erfindung ist daher, ein Verfahren bereitzustellen, mit dem die genannten Nachteile des Standes der Technik überwunden werden können.

Die vorliegende Erfindung beseitigt diese Nachteile in einem einfach zu installierenden und bedienenden, kostengünstigen und kontaktfreien Schlafüberwachungssystem. Dieses System ist sowohl für den klinischen als auch den Einsatz zu Hause geeignet und kann Schlafposition, Schlafphasen, pathologische Bewegungsmuster, z.B. Restless Leg Syndrom (RLS)/Periodic Leg Movement Syndrom (PLMS) als auch pathologische Atemstörungen, z.B. obstruktive und zentrale Schlafapnoe klassifizieren.

Das erfindungsgemäße Problem wird durch ein Verfahren gemäß Anspruch 1 gelöst. Vorteilhafte Ausgestaltungen der Erfindung sind in den Unteransprüchen angegeben und werden nachfolgend beschrieben.

Danach ist vorgesehen, dass das Verfahren zur berührungslosen Ermittlung von Atem- und Beinbewegungen schlafender Personen mit einem Tiefensensor mindestens die Schritte umfasst:
- Bereitstellen einer insbesondere vorverarbeiteten Zeitserie von Tiefenbildern des Tiefensensors, die einen Schlafplatz mit der schlafenden Person umfassen,
- Ermitteln und Quantifizieren zumindest eines Bewegungssignals, das aus Bewegungen mindestens eines Körperbereichs der schlafenden Person aus der Zeitserie der Tiefenbilder ermittelt wird, wobei der mindestens eine Körperbereich den Torso, insbesondere den Brustbereich der Person umfasst und/ oder wobei der mindestens eine Körperbereich den Beinbereich der Person umfasst.

Der Beinbereich umfasst die Beine und insbesondere auch die Füße möglichst engräumig. Die Bewegungen der Beine werden also durch das mindestens eine Bewegungssignal des Beinbereichs erfasst, und Atembewegungen werden durch das mindestens eine Bewegungssignal aus dem Torsobereich erfasst. Die Körperbereiche können gleichzeitig und aber auch getrennt voneinander erfasst werden. Insbesondere werden die Bewegungssignale für jeden Körperbereich separat bestimmt. Die Bewegungen des jeweiligen Körperbereichs entsprechen insbesondere den Bewegungen der Beine bzw. Bewegungen des Torsos, die durch das Atmen der Person hervorgerufen werden.

Ein Tiefensensor im Sinne der Erfindung ist insbesondere dazu ausgelegt, auch in Dunkelheit zu funktionieren also messfähig zu sein. Beispiele für einen solchen Tiefensensor sind eine aktive Infrarot Time of Flight Kamera (z.B. Kinect V2 von Microsoft), eine aktive structured light Infrarotkamera (Kinect V1 von Microsoft), sowie eine Infrarot Stereokamera mit Infrarotlampe.

Der Tiefensensor sollte mindestens ein field of view von 45 x 60 Grad mit einer Pixeldichte von 5 x 5 Pixel pro Grad aufweisen. Des Weiteren sollte der Messbereich des Tiefensensors mindestens Abstände zwischen 1 m und 3 m umfassen, damit das Mess-Setup eine komplette bildliche Abdeckung des Betts ermöglicht und gleichzeitig praktikabel bleibt. In diesem Messbereich sollte die Genauigkeit der räumlichen Auflösung des Tiefensensors 5 mm nicht unterschreiten.

Die zeitliche Auflösung des Tiefensensors (Bilder pro Sekunde / frames per second (fps)) sollte 30 fps nicht unterschreiten.

Zusätzlich ist es vorteilhaft, die Orientierung des Tiefensensors relativ zum Boden nach dem Aufbau insbesondere mit einem 3-Achsen Beschleunigungsmesser zu messen. Dies kann z.B. durch fest einen installierten Beschleunigungsmesser im Tiefensensor geschehen (so bei Kinect V2), durch einmalige Aufnahme durch den Benutzer mit Hilfe eines Smartphones oder einem sonstigen Computer und einer spezialisierten App oder zusätzlich am Tiefensensor angebrachte low-cost Beschleunigungsmesser, die per Bluetooth oder Wlan an einen Aufnahmerechner gekoppelt werden können.

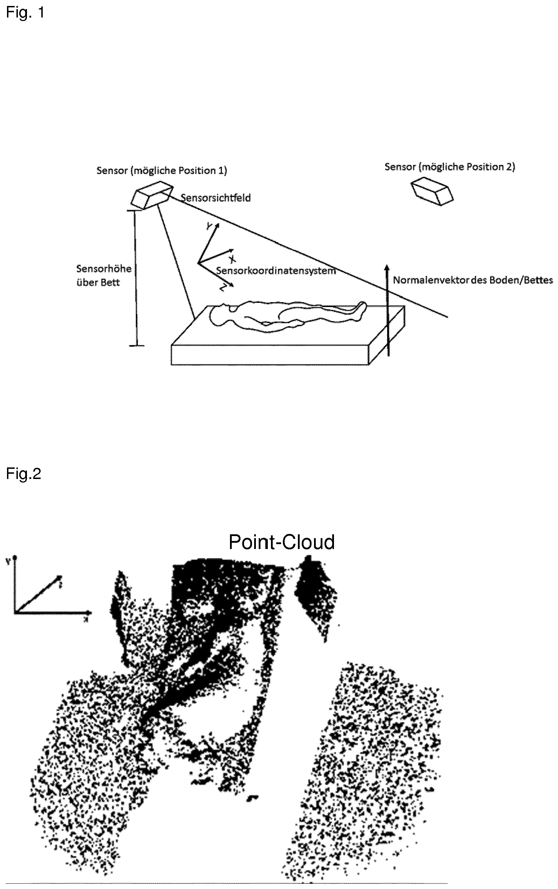

Der Tiefensensor sollte so ausgerichtet werden, dass er das Bett und die gesamte darin liegende Person von oben im Blick hat. Der Sensor muss nicht zentral über dem Bett angebracht sein, sondern kann von einer beliebigen Seite auf das Bett schauen. Der maximale Abstand des Sensors zum Bett sollte dabei höchstens 4 m betragen (siehe Fig. 1).

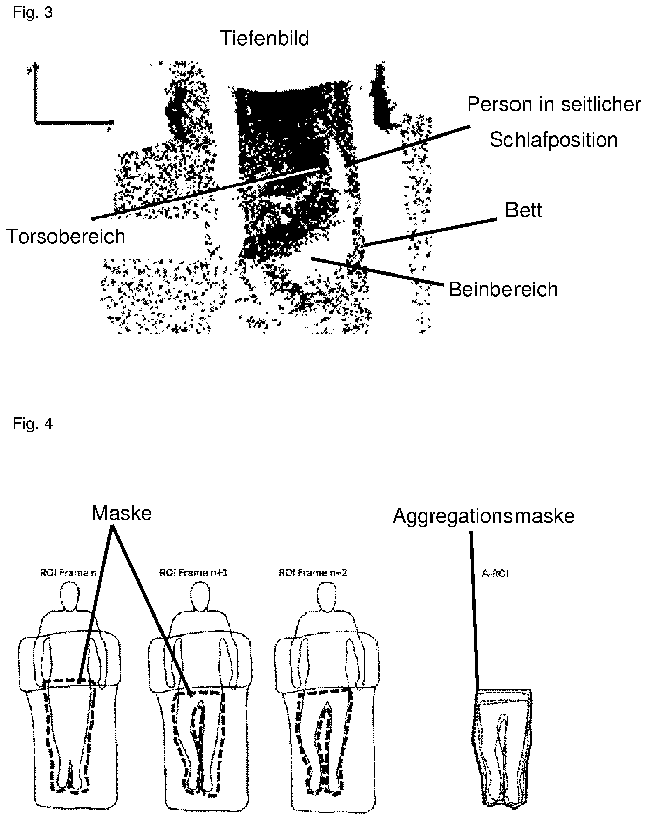

Die Messdaten des Tiefensensors werden beispielsweise in Form von Tiefenbildern von diesem geliefert, in denen insbesondere jeder Pixel einen Abstandswert darstellt. Da jedem Pixel auch ein Richtungswinkel zugeordnet ist, kann aus der Kombination dieser Informationen die räumliche Position des Pixel-Messpunktes in Relation zum Tiefensensor errechnet werden. Die Gesamtheit der Pixel-Messpunkte, die aus einem Tiefenbild errechnet werden, wird im Folgenden als Point-Cloud (dt. Punktwolke) bezeichnet (siehe Fig. 2+3). Im Normalfall bieten Tiefensensoren die Transformation von Pixel- und Winkelinformation zu Raum-Punkt Information als Funktion in mitgelieferten Software Development Kits an, welche die Winkeleigenschaften des Tiefensensors akkurat abbilden. Tiefenbilder können auch eine andere Form als die gerade beschriebene aufweisen, umfassen aber zumindest eine dreidimensionale Raumpunktinformation einer Vielzahl von Raumpunkten.

Es bietet sich an, die gemessenen Tiefensensordaten vor Verarbeitung durch die hier offenbarten erfindungsgemäßen Verfahren vorzubereiten. Diese Vorverarbeitung kann auf verschiedene Arten und Weisen erfolgen. Im Folgenden werden beispielhaft einige Vorverarbeitungsschritte aufgezeigt, die vollständig oder nur teilweise auf die Tiefenbilder angewandt werden können, um Tiefenbilder ausreichender Qualität zu erlangen.
1. Die von der jeweiligen Tiefensensorposition und Ausrichtung abhängigen Point-Cloud Koordinaten werden so rotiert, dass der Normalvektor des Bodens und/ oder des Bettes nach der Rotation parallel zur Z-Achse des Tiefensensorkoordinatensystems liegt, d.h. senkrecht auf den Sensor des Tiefensensors zeigt.
2. Die gesamte Point-Cloud wird so verschoben, dass der Mittelpunkt der Point-Cloud in einem Nullpunkt des somit normalisierten Koordinatensystems des Sensors liegt (im Folgenden auch als Normalkoordinatensystem bezeichnet). Durch diese Operationen wird die Point-Cloud im Koordinatensystem des Sensors zentriert und die Boden- bzw. Bettebene parallel zur X-Y-Ebene im Normalkoordinatensystem des Sensors ausgerichtet. Dadurch ist die Position der Point-Cloud nicht mehr beeinflusst durch aufbauspezifische Sensorwinkel des Tiefensensors.
3. Die Point-Cloud wird so auf die X-Y-Ebene (also die Bett-Ebene) des Normalkoordinatensystems abgebildet, dass eine zwei-dimensionale Tiefenmatrix entsteht, bei der jeder Eintrag dem Abstand vom Boden in beispielsweise Millimeter entspricht. Diese Matrix stellt ebenso ein Tiefenbild im Sinne der Erfindung dar, in dem jeder Pixel einen Tiefenwert repräsentiert. Damit werden insbesondere durch die Position des Tiefensensors bedingte perspektivische Verzerrungen ausgeglichen und die Tiefeninformation im Tiefenbild ist nicht mehr abhängig von der Entfernung zum Sensor.
4. Der Teil des Tiefenbildes, der das Bett umfasst, wird mit Hilfe einer Region of Interest Segmentierungsmethode erkannt und das Tiefenbild wird auf den Bereich, der das Bett enthält, zugeschnitten. Dies kann beispielsweise unter Zuhilfenahme einer sogenannten Iterative Selection Thresholding Method [1] geschehen, um übriggebliebene Bereiche außerhalb des Bettes, z.B. den Boden, zu entfernen.
5. Es wird ein Boxfilter auf der Zeitserie der Tiefenbilder angewendet, der jeden Pixel eines Tiefenbildes mit dem Mittelwert des gleichen Pixels im vorgehenden, aktuellen und folgenden Tiefenbild ersetzt. Dieser Schritt ist insbesondere geeignet, um Rauschen zwischen Tiefenbildern zu verringern.
6. Es wird ein Medianfilter [2] auf jedes Tiefenbild der Zeitserie angewendet, insbesondere um Rauschen zu unterdrücken und gleichzeitig Kanten im Tiefenbild zu bewahren. Als Größe des Medianfilters kann beispielsweise 3 Pixel x 3 Pixel gewählt werden.

Die vorgenannten Schritte 1 bis 6 sind optionaler Natur, doch wirkt sich insbesondere eine Vorverarbeitung der Tiefensensordaten gemäß der Schritte 1 bis 4 vorteilhaft auf die Performance des erfindungsgemäßen Verfahrens aus. Diese Schritte sind auch in Fig. 2 bis 3 dargestellt.

Weiterhin kann während des Verfahrens insbesondere ein Mittelwert der Orientierung des Tiefensensors insbesondere über die Dauer der Aufnahme berechnet werden und anhand dieser Orientierung die Tiefenbilder nach dem oben beschriebenen Verfahren vorverarbeitet werden. Die Tiefensensor Orientierung ist beispielsweise ein Vektor, der die Neigung des Tiefensensors relativ zum Boden oder zur Gravitationsrichtung angibt. Diese sollte mindestens einmalig je Messung erfasst werden. Je nach Rauschverhalten des genutzten Orientierungssensors ist die Bildung des Mittelwerts dieser Vektoren als mittlere Orientierung sinnvoll. Ebenso lässt eine fortlaufende Auswertung der Orientierung zu, eventuelle Positionsänderungen des Tiefensensors während der Messung, z.B. durch unsachgerechte Anbringung des Tiefensensors, zu erkennen und zu kompensieren.

Eine Zeitserie ist dabei die insbesondere fortlaufende Folge der Tiefenbilder des Tiefensensors, also beispielsweise eine Bildfolge mit einer bestimmten Framerate (siehe oben). Die Zeitserie kann auch nur eine Teilmenge der aufgenommenen Tiefenbilder des Tiefensensors umfassen, so dass eine geringere Datenmenge pro Zeiteinheit anfällt. Dies ist insbesondere vorteilhaft, wenn die Rechenleistung begrenzt ist, oder die Dauer der Segmentierungsalgorithmen / Klassifikationsalgorithmen eine niedrigere Datenrate notwendig macht und/oder wenn eine solch hohe Datenrate nicht benötigt wird. Die Framerate liegt üblicherweise bei 30 fps.

Es wird angemerkt dass für alle Mittelwerte, die im Zusammenhang mit der Erfindung erwähnt werden, soweit nicht explizit ausgeschlossen, alle geeigneten Mittelwertmaße in Frage kommen, wie beispielsweise der arithmetische, oder geometrische Mittelwert, ein Median, oder ein in sonstiger Weise geeigneter, insbesondere gewichteter Mittelwert.

Gemäß einer Ausführungsform wird zum Ermitteln des mindestens einen Bewegungssignals des mindestens einen Körperbereichs der Person in jedem Tiefenbild der Zeitserie eine Maske umfassend den jeweiligen Körperbereich ermittelt und diesem Körperbereich zugeordnet.

Dies ermöglicht in vorteilhafter Weise eine bessere Signalqualität, da Raumbereiche, in denen kein Nutzsignal zu erwarten ist, nicht in das Bewegungssignal eingehen.

Eine Maske, ist beispielsweise ein geschlossenes Polygon, das einen gewissen Bildbereich des Tiefenbildes umschließt.

An verschiedenen Stellen des Verfahrens kann eine Segmentierung der Tiefenbilder zum Einsatz kommen, um die jeweilige Maske bzw. den gesuchten Bildbereich zu generieren bzw. zu identifizieren. Beispielsweise bei der
- Erkennung des Bettumrisses und der Lage des Kopf- und Fußendes des Bettes,
- Erkennung der schlafenden Person,
- Erkennung des Brust-/ Torsobereichs insbesondere unter einer Bettdecke,
- Erkennung des Beinbereichs bzw. der Beine der schlafenden Person insbesondere unter einer Bettdecke.

Für eine solche Segmentierung wird jeweils ein Trainingsset von Tiefenbildern erstellt, in welchen die entsprechenden korrekten Masken hinterlegt und mit Klassen-Labeln annotiert sind, also für beispielsweise Kopfende und Fußende, Brust, Beine, aber z.B. auch Kopf und Arme. Der Torsobereich und der Beinbereich leiten sich von der Erkennung der gesamten Person ab und werden insbesondere anschließend an die Erkennung der gesamten Person durchgeführt.

Ein möglicher Segmentationsalgorithmus der mit solchen Tiefendaten trainiert und zur Klassifikation genutzt werden kann ist beispielsweise das Random Forest basierte, "pixel-wise body part labeling" entsprechend Shotton [8]. Dieses benötigt umfangreiche Trainingssets, um akzeptable Ergebnisse zu liefern.

Ein anderer möglicher Segmentationsalgorithmus ist die Objekterkennung entsprechend Felzenszwalb [9], welcher mit weniger Trainingsaufwand sogenannte bounding boxes für die einzelnen Masken liefert, aber entsprechend weniger akkurat deren Form abbildet als ein Pixel-basierter Masken Ansatz wie bei Shotton. Dies bewirkt, dass z.B. darauf aufbauende Bewegungsextraktionen entsprechend gedämpft oder verrauschter sind. Nachteilhaft ist bei diesem Algorithmus insbesondere, dass er für RGB Bilder optimiert ist, und in der Auswertung einen hohen Rechenaufwand erfordert.

Gemäß einer weiteren Ausführungsform der Erfindung werden die zugeordneten Masken zeitbereichsweise zu einer Serie von Aggregationsmasken zusammengefasst, wobei die jeweilige Aggregationsmaske den jeweiligen Tiefenbildern des jeweiligen Zeitbereichs zugeordnet wird (siehe z.B. Fig. 4).

Gemäß dieser Ausführungsform der Erfindung wird also über einen bestimmten Zeitbereich eine unveränderte Maske, nämlich die Aggregationsmaske, auf die Tiefenbilder aus diesem Zeitbereich gelegt. Die Aggregationsmaske kann insbesondere aus der Vereinigung aller Masken aus dem jeweiligen Zeitbereich ermittelt werden.

Dieser Zeitbereich kann entweder von vordefinierter Länge sein - beispielsweise eine Minute - oder er kann über die Dauer der gesamten Messung individuell dynamisch festgelegt werden. Im letzteren Fall erstrecken sich die Aggregationsmasken also ggf. über unterschiedlich viele Tiefenbilder.

Falls die Zeiträume, über die sich die Aggregationsmasken erstrecken, nicht von vordefinierter Länge sind, also dynamisch festgelegt werden, kann das folgende Verfahren zur Bestimmung der Zeiträume - auch Zeitintervalle genannt - angewandt werden:
- Berechnung mindestens eines Bewegungssignals, also insbesondere das Ermitteln von Bewegungskenngrößen für den gesamten Körper der Person für die Tiefenbilder Zeitserie. Hierbei wird das mindestens eine Bewegungssignal aus dem gesamten Aufnahmebereich des Tiefenbildes, der das Bett umfasst, ermittelt. Das mindestens eine Bewegungssignal kann dabei analog zu der weiter unten aufgeführten Ermittlung des mindestens einen Bewegungssignals des Beinbereichs erfolgen.
- Wie bereits oben beschrieben, werden die Größe und die Position der Masken in jedem Tiefenbild insbesondere für den Beinbereich ermittelt. Als Maß der Größe einer Maske kommen dabei die eingeschlossene Fläche, ein Außen- oder Innendurchmesser der Maske oder andere geeignete Parameter in Frage.
- Das Ende eines Zeitraums und damit auch der Beginn eines darauffolgenden Zeitraums, für den die jeweilige Aggregationsmaske aus der Aggregation der Masken ermittelt wird, wird durch die Amplitude des ermittelten Bewegungssignals des gesamten Körpers oder durch eine maximale Änderung von Position und/ oder Größe von aufeinanderfolgenden Masken bestimmt. Wenn die Amplitude des Bewegungssignals beispielsweise die drei-fache Standardabweichung des Bewegungssignals des betreffenden angebrochenen Zeitbereichs überschreitet, wird das angebrochene Zeitintervall beendet und ein neues Zeitintervall gestartet. Die in dem umfassten Zeitbereich ermittelten Masken werden zu der Aggregationsmaske zusammengefügt und eine neue Aggregationsmaske für die Masken im darauffolgenden gestarteten Zeitintervall bestimmt. Ebenso werden die aufeinanderfolgenden Masken solange zum gleichen Zeitintervall gezählt, bis die Position und/ oder die Größe der aktuell betrachteten Maske mehr als z.B. 20% von der ersten Maske des gleichen Zeitintervalls abweicht. In diesem Fall startet die aktuelle Maske ein neues Zeitintervall und der Prozess beginnt von vorn. Zeitintervalle und damit die Aggregation der Masken zu der jeweiligen Aggregationsmaske "wachsen" also so lange, bis eine zu starke Änderung der Maskenposition und/ oder Maskengröße ein neues Intervall starten lässt.
- Beide Kriterien, also das Bewegungssignal des Körpers und die Maskenpositions- und Größenänderung sind einzeln hinreichend, um eine Intervallgrenze festzulegen; es muss also nur ein Kriterium erfüllt sein.

Gemäß einer weiteren Ausführungsform der Erfindung wird für jedes Tiefenbild aus einem Bildbereich, der innerhalb der jeweiligen zugeordneten Aggregationsmaske liegt, insbesondere aus den Werten der von der Aggregationsmaske umschlossenen Pixeln des Tiefenbildes, mindestens eine Bewegungskenngröße für den mindestens einen Körperbereich ermittelt und dem jeweiligen Tiefenbild zugeordnet.

Diese Reduktion auf spezifische Bewegungskenngrößen ist vorteilhaft, da so in geeigneter Weise Parameter bestimmt werden, deren Werte und Wertänderungen mit den Bewegungen des jeweiligen Köperbereichs korrelieren und so ein trainierter Classifier eines maschinellen Lernalgorithmus diese Kenngrößen verwenden kann, um eine Klassifikation vorzunehmen.

Gemäß einer weiteren Ausführungsform der Erfindung ist vorgesehen, dass die mindestens eine Bewegungskenngröße, wenn das mindestens eine Bewegungssignal aus dem Torsobereich ermittelt wird, ein Mittelwert der Tiefendaten, wie beispielsweise das arithmetische oder geometrische Mittel, oder ein Median oder ein in sonstiger Weise geeigneter Mittelwert, über dem von der Aggregationsmaske umfassten Bildbereich des jeweiligen Tiefenbildes ist, wobei das zur Atembewegung zugehörige mindestens eine Bewegungssignal die zeitliche Folge der Bewegungskenngröße umfasst.

Die Bewegungskenngröße zeigt insbesondere an, ob sich der von der Aggregationsmaske umschlossene Bereich im Mittel hebt oder senkt, insbesondere welche mittlere Höhe er in dem jeweiligen Tiefenbild aufweist. Die Ermittlung der Bewegungskenngröße umfasst insbesondere die Bestimmung der mittleren Pixeltiefe, d.h. das arithmetische Mittel der Höhe aller Punkte über dem Boden in der Aggregationsmaske.

Die Bewegungskenngröße wird insbesondere für jedes Tiefenbild ermittelt, zu dem die jeweilige Aggregationsmaske zugeordnet ist. Für jede Aggregationsmaske kann also eine Zeitspur für jede der mindestens einen Bewegungskenngröße ermittelt werden, wobei jede Bewegungskenngröße ein Bewegungssignal begründet. Alle Zeitspuren zusammengenommen spiegeln den gesamten Atembewegungsverlauf über den Messzeitraum wieder.

Gemäß einer weiteren Ausführungsform der Erfindung, wird das zur Atembewegung zugehörige mindestens eine Bewegungssignal mit Atembewegungs-Referenzmustern verglichen und kann so insbesondere auf pathologische Merkmale untersucht werden.

Diese bestimmten Atembewegungs-Referenzmuster werden zur Detektion von pathologischen Atembewegungen wie z.B. Apnoe verwendet und ggf. mit einem Pattern-Matching Algorithmus mit den ermittelten Bewegungssignalen verglichen.

Alternativ oder ergänzend wird zur Detektion von pathologischen Atembewegungen eine Signalhüllkurve des Atemsignals, also insbesondere des Bewegungssignals der Atembewegungen, berechnet. Hierzu werden beispielsweise die Maxima und Minima des Atemsignals in geeigneter Weise bestimmt. Anschließend werden sowohl Maxima als auch Minima in geeigneter Weise zu jeweils einem Signal verbunden, insbesondere durch Nichtberücksichtigen, Auslassen oder Entfernen, der Teile des Atemsignals, die keine Maxima und Minima umfassen. Das so erzeugte Signal ergibt die Signalhüllkurve. Über die Amplitude oder mittlere Höhe der Signalhüllkurve des Atemsignals kann über geeignete Schwellwerte beispielsweise festgestellt werden, ob, wann und wie lange eine pathologische Atembewegung stattfindet.

Eine besondere Herausforderung beim Ermitteln des Bewegungssignals der Atembewegungen stellt hierbei die teilweise Verdeckung des Bauches und des Brustkorbes durch die Bettdecke dar. Vorteilhaft ist es daher, die zusätzlichen Informationen der Körperlage der Person (also die jeweilige Schlafposition) zu ermitteln um eine zuverlässige Extraktion des Atemumfangs möglich zu machen, da z.B. die verwendeten Referenzmuster zur Erkennung von Atembewegungsstörungen davon abhängen.

Gemäß einer weiteren Ausführungsform der Erfindung ist vorgesehen, dass die mindestens eine Bewegungskenngröße, wenn das mindestens eine Bewegungssignal aus den Beinbewegungen ermittelt wird, ein Mittelwert der Tiefendaten über dem von der Aggregationsmaske umfassten Bildbereich des jeweiligen Tiefenbildes ist und/ oder wobei die mindestens eine Bewegungskenngröße eine Größe, beispielsweise eine umschlossene Fläche bzw. Pixelzahl, ein Umfang, ein Außen- oder Innendurchmesser, eine Position und/ oder eine Bewegungsrichtung der dem jeweiligen Tiefenbild zugeordneten Maske ist, und/ oder wobei die mindestens eine Bewegungskenngröße eine Position und/ oder eine Bewegungsrichtung von Merkmalen, beispielsweise besonders hoher Punkte oder Kanten, innerhalb der jeweiligen Maske ist, wobei die Position und/ oder die Bewegungsrichtung besagter Merkmale insbesondere durch Verwendung eines optical flow Algorithmus ermittelt wird, wobei das zur Beinbewegung zugehörige mindestens Bewegungssignal die zeitliche Folge der mindestens einen Bewegungskenngröße umfasst und wenn mehrere Bewegungskenngrößen ermittelt werden, jede zeitliche Folge einer solchen Bewegungskenngröße einem Bewegungssignal zugeordnet wird.

Da Beinbewegungen oftmals die mittlere Höhe des Tiefenbildes nicht oder nur wenig ändern, werden insbesondere mehrere Bewegungskenngrößen bestimmt, die mit einer Beinbewegung korrelieren - so deutet eine laterale Verschiebung der Masken, die den Beinbereich umfassen, auf eine Bewegung der Beine hin. Daher ist die Position und/ oder die Bewegungsrichtung der konsekutiven Masken eine geeignete Bewegungskenngröße bei der Ermittlung der Beinbewegung.

Gemäß einer weiteren Ausführungsform der Erfindung ist vorgesehen, dass für jedes zur Beinbewegung zugehörige mindestens eine Bewegungssignal, Spitzen des jeweiligen Bewegungssignals mit einem Spitzendetektionsverfahren ermittelt werden, und wobei insbesondere anhand der Verteilung und der Höhe der Spitzen in dem jeweiligen mindestens einen Bewegungssignal, Bewegungen der Beine detektiert und klassifiziert werden, insbesondere wird der Periodic Limb Movement Index Score (PLMI) als durchschnittliche Anzahl der Periodic Limb Movements pro Stunde ermittelt.

Die Werte der Höhe der Spitzen liegen, insbesondere für das Mediangradiententiefensignal der Beine, zwischen 0.09 und 1.0 mm/frame oder - 0.15 und -1.0 mm/frame bei negativer Auslenkung.

Das Mediangradiententiefensignal ist dabei durch die zeitliche Ableitung des Mediantiefensignals gegeben, also die Rate der Änderungen des Medians pro Frame der Beinmaske.

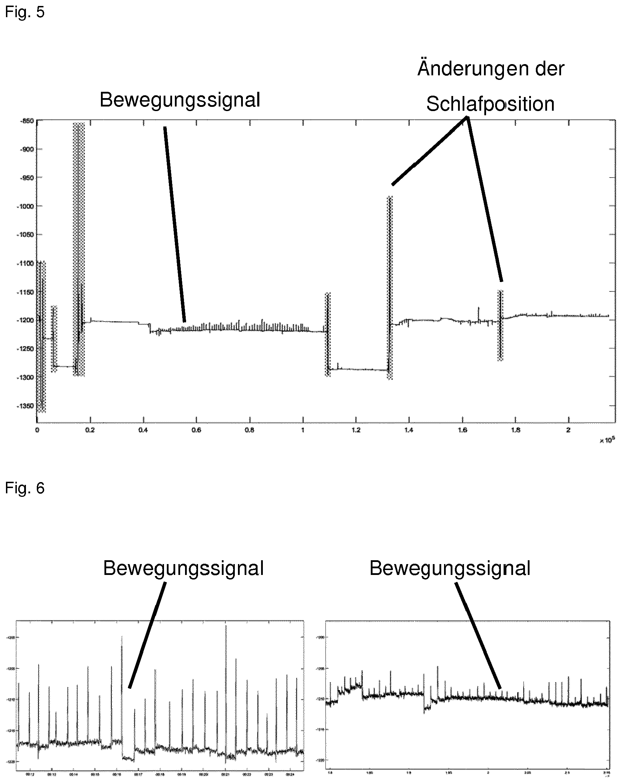

Es ist auch möglich und optional vorgesehen, das aus der mindestens einen Bewegungskenngröße ermittelte mindestens eine Bewegungssignal zu filtern, bevor eine Klassifikation der Spitzenverteilung vorgenommen wird oder die Spitzenerkennung durchgeführt wird, und zwar insbesondere so, dass Abschnitte mit normalen Bewegungen herausgefiltert werden. Normale Bewegungen treten natürlich im Schlaf auf und können z.B. eine Änderung der Schlafposition oder-Schlafhaltung sein (z.B. Fig. 5). Im Gegensatz zu pathologischen Bewegungen sind diese nicht periodisch und nur auf einen kurzen zeitlichen Abschnitt (z.B. 2 Sekunden) beschränkt. Normale Bewegungen zeigen sich deutlich im Beinbewegungssignal und lassen sich durch Grenzwerte von z.B. dem 5-fachen der Standardabweichung des Bewegungssignals detektieren.

Auch eine gleichzeitig ermittelte Schlafposition kann herangezogen werden, um das mindestens eine Bewegungssignal der Beine oder des Torsos von Fehlsignalen zu befreien, indem das mindestens eine Bewegungssignal mit der Schlafposition verglichen wird und entsprechend korrigiert wird.

Die Detektion der einzelnen Beinbewegungen erfolgt durch Spitzendetektion auf dem Bewegungssignal, wobei detektierte Spitzen insbesondere einen Abstand von mindestens 150 Tiefenbildern, insbesondere bei 30 fps und eine Prominenz von insbesondere mehr als 1.5 mm aufweisen sollten. Diese Werte sind im klinischen Kontext üblich und können auch hier verwendet werden. Der verwendete Algorithmus für die Spitzendetektion in Abhängigkeit von Abständen und Prominenz ist beispielsweise beschrieben unter http://de.mathworks.com/help/signal/ref/findpeaks.html?requestedDomain=www.mat hworks.com

Gemäß einer weiteren Ausführungsform der Erfindung wird das mindestens eine Bewegungssignal anschließend in nicht überlappende Abschnitte von beispielsweise 30 Sekunden Länge eingeteilt. Diese Abschnitte können auch eine andere Länge haben, wichtig dabei ist, dass das mindestens eine Bewegungssignal in diesem Abschnitt annähernd stabil und stationär ist (insbesondere keine mittlere Steigung oder starke Sprünge aufweist). Auf jeden dieser Abschnitte wird ein Rauschfilter angewandt. Beispielsweise kann in jedem dieser Abschnitte die Standardabweichung oder ein anderes Maß der Varianz des Bewegungssignals bestimmt werden und sodann diejenigen detektierten Spitzen innerhalb eines solchen Abschnittes verworfen werden, deren Signalamplitude unterhalb der dreifachen Standardabweichung des Signalabschnitts liegen. Diese Methode dient der Verbesserung der Ergebnisse bei starkem Rauschverhalten im Signalabschnitt. Andere Verfahren zur Rauschminderung oder zur Entscheidung welche Spitzen valide sind, könnten auf Frequenzanalyse des Abschnittes oder auf anderen Grenzwerten basieren.

In jedem Fall können sodann anhand der detektierten und ggf. gefilterten Spitzen, welche Einzelbewegungen entsprechen, pathologische Bewegungsmuster identifiziert werden. Diese bestehen insbesondere aus periodischen Wiederholungen der Beinbewegungen im Abstand von 5 bis 90 Sekunden. Wenn Beinbewegungen aus verschiedenen Bewegungskenngrößen extrahiert werden, können diese aggregiert werden. Für eine weitere Analyse können alle Bewegungssignale aller Bewegungskenngrößen verwendet werden. Pathologische Beinbewegungen zeichnen sich z.B. dadurch aus, dass sie sich regelmäßig (periodisch) im Abstand von 5 bis 90 Sekunden wiederholen. Zur Detektion dieser periodischen Wiederholungen kann z.B. eine fensterbasierte Frequenzanalyse oder eine Analyse der zeitlichen Abstände zwischen aufeinanderfolgenden Bewegungen genutzt werden. Eine weitere Option zur Ermittlung der Periodizität oder quasi-Periodizität ist die Verwendung eines Korrelationsalgorithmus, wie beispielsweise einer Autokorrelation oder einer Wavelet-Analyse.

Gemäß einer weiteren Ausführungsform der Erfindung erfolgt die Ermittlung der Maske des mindestens einen Körperbereichs mit einem, insbesondere überwachten, maschinellen Lernalgorithmus, insbesondere einem Segmentationsalgorithmus. Geeignete Segmentationsverfahren sind bereits oben erwähnt worden.

Das erfindungsgemäße Problem wird weiterhin durch ein Verfahren zur berührungslosen Klassifikation von Schlafphasen einer schlafenden Person mit einem Tiefensensor gelöst, welches mindestens die folgenden Schritte aufweist:
- Zur Verfügung stellen einer insbesondere vorverarbeiteten Zeitserie von Tiefenbildern umfassend Tiefendaten des Tiefensensors, wobei die Tiefenbilder einen Schlafplatz mit der Person umfassen,
- Verarbeiten der Tiefenbilder und Ermitteln der Schlafphase durch ein künstliches, neuronales Netzwerk, insbesondere durch ein 3D Convolutional oder Long short-term memory (LSTM) Neural Net [10], und/ oder
- Ermitteln der Schlafphase, wobei Schlafphasenkenngrößen der Tiefenbilder ermittelt werden und wobei ein trainierter Classifier eines maschinellen Lernens Algorithmus die Schlafphase anhand der zur bereitgestellten Schlafphasenkenngrößen ermittelt.

Schlafphasen können beispielsweise in wach, eine RapidEyeMovement-Phase, und N1, N2, N3, wobei N3 dem Tiefschlaf entspricht, klassifiziert werden. Entsprechend ermittelt das erfindungsgemäße Verfahren einen Teil oder alle dieser Schlafphasen und gibt diese aus.

Das erfindungsgemäße Verfahren ist dabei besonders vorteilhaft, da es die Schlafphase insbesondere berührungsfrei ermittelt und so beispielsweise im Gegensatz zu bekannten Verfahren (EEG-Messung), die mit Elektroden-Verkabelung verbunden sind, deutlich weniger invasiv ist und daher das Schlafverhalten der Person nicht stört.

Gemäß einer Ausführungsform der Erfindung werden, wenn das Ermitteln der Schlafphase durch einen trainierten Classifier eines maschinellen Lernen Algorithmus erfolgt, die Schlafphasenkenngrößen mit mindestens den folgenden Schritten ermittelt:
- Ermitteln einer Zeitserie, die insbesondere eine niedrigere Bildrate aufweist, als die obigen Verfahren zur Ermittlung von Bewegungskenngrößen, von Differenztiefenbildern mit Differenztiefendaten, wobei ein Differenztiefenbild aus der Differenz der Tiefendaten zweier aufeinanderfolgender Tiefenbilder der Zeitserie ermittelt wird,
- Ermitteln eines dem jeweiligen Differenztiefenbild zugeordneten gemittelten Bewegungswertes, wobei der gemittelte Bewegungswert einen Mittelwert, wie beispielsweise einen arithmetischen oder geometrischen Mittelwert oder einen Median, insbesondere aller Differenztiefendaten des jeweiligen Differenztiefenbildes umfasst,
- Ermitteln eines zeitgemittelten Bewegungsbildes, wobei die Pixelwerte des zeitgemittelten Bewegungsbildes den insbesondere absoluten Abweichungen der Differenztiefendaten der Differenztiefenbilder entsprechen,
- Ermitteln von Schlafphasenkenngrößen für das zeitgemittelte Bewegungsbild und für die gemittelten Bewegungswerte, wobei die Schlafphasenkenngrößen einen arithmetischen und/ oder einen geometrischen Mittelwert, und/ oder einen Median der entsprechenden Werte umfassen, und/ oder den höchsten und/ oder den niedrigsten Wert, und/ oder einen Mittelwert über die n-höchsten Werte und/ oder m-niedrigsten Werte, wobei n und m eine vorbestimmte Zahl, insbesondere 100 ist, umfassen, wobei die besagten Schlafphasenkenngrößen für das gesamte zeitgemittelte Bewegungsbild jeweils auch bildbereichsweise ermittelt werden, wobei das zeitgemittelte Bewegungsbild dazu in insbesondere gleichförmige und gleichgroße Bereiche unterteilt wird, die das gesamte zeitgemittelte Bewegungsbild umfassen und wobei insbesondere für jeden Bereich auch die Varianz und die größte principal component der umfassten Werte des zeitgemittelten Bewegungsbildes ermittelt wird und von den Schlafphasenkenngrößen umfasst ist.

Die ermittelte Zeitserie weist insbesondere eine Länge von 30 Sekunden auf, was 900 Tiefenbildern bei 30 fps entspricht. D.h. die Schlafphasenermittlung über die gesamte Messdauer umfasst insbesondere mehrere solcher Zeitserien. Für jede dieser Zeitserien kann das erfindungsgemäße Verfahren durchgeführt werden. Die Länge der Zeitserie von 30 Sekunden ist insbesondere so gewählt, da die Daten eines Referenz Polysomnographieverfahrens üblicherweise diese zeitliche Auflösung haben.

Es können auch kürzere Zeitserien gewählt werden, z.B. 15 Sekunden, 10 Sekunden, oder 5 Sekunden. Dies erhöht allerdings die benötigte Zeit und den Speicherbedarf für das Training des Classifiers, da mehr Trainingsdaten erzeugt werden. Das erfindungsgemäße Verfahren kann bei einer Zeitserie von 900 Tiefenbildern beispielsweise wie folgt ausgeführt werden:
a. Es werden 899 Differenztiefenbilder von aufeinanderfolgenden Tiefenbildern berechnet. Für jedes Differenztiefenbild wird beispielsweise der durchschnittliche Pixelwert berechnet, also die Summe aller Pixel normiert durch die Anzahl der Pixel im Differenztiefenbild. Dadurch ergeben sich 899 skalare Werte, die gemittelten Bewegungswerte.
b. Der Pixelwert des zeitgemittelten Bewegungsbildes an der Stelle (i,j) ist die Summe der absoluten Differenzen der Pixelwerte an der Stelle (i,j) von konsekutiven Tiefenbildern, normiert durch die Anzahl der aufsummierten Werte, hier 899.
c. Für die gemittelten Bewegungswerte werden Median, Mittelwert, Maximum, Minimum und geometrischer Mittelwert berechnet.
d. Für das zeitgemittelte Bewegungsbild werden Mittelwert, Maximum, Median aller Werte und der Mittelwert der höchsten 100 Pixelwerte berechnet.
e. Über das zeitgemittelte Bewegungsbild wird ein gleichmäßiges Raster gelegt, z.B. mit einer Kantenlänge von 1/10 der Bettlänge, was bei einem Kinect V2 Sensor etwa 40x40 Pixeln entspricht. Für jedes dieser Rasterfelder werden Mittelwert, Median, Maximum, Minimum, Varianz und die größte principal component als Ergebnis einer Principal Component Analysis auf den Pixeln des Feldes mit zugehöriger Varianz berechnet und insbesondere abgespeichert.

Die so berechneten Schlafphasenkenngrößen aus c, d und e werden konkateniert und zusammen mit vorhandenen Annotationen über die Schlafphase aus einem Polysomnographie Referenz System benutzt, um einen Classifier mit Methoden des machine learning zu trainieren.

Mögliche Verfahren des maschinellen Lernens und Classifier sind beispielsweise RUSBoost [4] und Enscheidungsbäume [5] oder Subspace [6] KNNs.

Der so trainierte Classifier kann anschließend benutzt werden, um Zeitintervalle mit extrahierten Schlafphasenkenngrößen von Personen nach Schlafphasen zu klassifizieren. Dafür durchlaufen neue Aufnahmen die gleichen Preprozessing- und Schlafphasenkenngrößenermittlungsschritte. Anschließend werden die extrahierten Schlafphasenkenngrößen mit dem trainierten Classifier klassifiziert.

Künstliche neuronale Netze (KNN oder NN) sind eine Methode des maschinellen Lernens, angelehnt an die Funktionsweise von echten neuronalen Netzen [11]. Hierbei werden einfache Bausteine, die künstlichen Neurone, zu komplexeren Netzwerken zusammengesetzt. Diese Netzwerke können anhand von Trainingsdaten (Input und gewünschter Output) trainiert werden, um für spätere, noch nicht vorher gezeigte Inputs die gewünschten Outputs zu erzeugen [12].

Meist werden die künstlichen Neurone in solchen Netzen in unterschiedlichen Schichten organisiert, wobei die erste Schicht (layer) als input layer, die letzte Schicht als output layer bezeichnet wird. Alle dazwischenliegenden Schichten werden als hidden layers bezeichnet. Netze mit mehr als einem hidden layer werden als sog. deep neural networks (DNN) bezeichnet [13].

Convolutional neural networks (CNN) bezeichnen eine spezielle Architektur von DNNs, die insbesondere in der Bildklassifikation, Objektdetektion, Handschrifterkennung etc. eingesetzt werden [14]. Long short-term memory networks (LSTM) gehören zur Klasse der recurrent neural networks (RNN). RNNs leiten ihren den Output zurück in sich selbst und benutzen ihn, zusammen mit dem nächsten Input, zur Berechnung des nächstens Outputs. LSTMs beinhalten außerdem eine spezielle Art von künstlichen Neuronen, um eine Art "Erinnerung" an die letzten Eingaben zu haben [15].

Alternativ zu letztgenannter Ausführungsform können auch direkt Deep Neural Networks zur Klassifikation eingesetzt werden. Insbesondere bei steigender Anzahl der Trainingsdaten ist dieser Ansatz sinnvoll, da hier zwar höhere Rechenkapazität für das Training benötigt wird, aber damit auch eine weitere Verbesserung der Klassifikationsergebnisse erzielt werden kann. Konkret kann ein 3D Convolutional Neural Net (3D CNN) eingesetzt werden, welches Tiefenbildsequenzen von z.B. 30 Sekunden oder 900 Frames verarbeitet und daraufhin die darin enthaltene Schlafphase klassifiziert. Ein möglicher Aufbau eines solchen 3D CNN ist beschrieben in [7].

Alternativ kann auch ein LSTM zur Klassifikation der Schlafphasen genutzt werden. Eine Beispielkonfiguration ist beispielsweise durch das Bereitstellen eines input layers gegeben, der dazu ausgebildet ist, Bilder der Größe 25x50 Pixel in Graustufen entgegen zu nehmen, bzw. der besagte Bilder entgegen nimmt. Weiterhin wird ein ein hidden layer aus LSTM Neuronen der Größe 16 sowie ein output layer der Größe 5 bereitgestellt, um 5 verschiedene Schlafphasen unterscheiden zu können. Es werden 1000 Bilder im Abstand von 10 Frames, also ca. 0.33 Sekunden, zu einer Sequenz zusammengefasst. Als Aktivierungsfunktion kann z.B. die Sigmoid-Funktion verwendet werden. Als Optimierungsfunktion kann z.B. Adam [17] verwendet werden. Als Fehlermaß wird der mean squared error (MSE) verwendet.

Wenn solche Neural Networks ohne Schlafphasenkenngrößenermittlung verwendet werden, können die Tiefenbilder der Zeitserie, z.B. wie oben 900 Frames, direkt zur Klassifikation in das NN gegeben werden, welches die Klassifikation der Schlafphasen vornimmt.

Das erfindungsgemäße Problem wird weiterhin durch ein Verfahren zur berührungslosen Klassifikation einer Schlafposition einer schlafenden Person mit einem Tiefensensor, zumindest aufweisend die Schritte gelöst:
- Zur Verfügung stellen einer insbesondere vorverarbeiteten Zeitserie von Tiefenbildern des Tiefensensors, die einen Schlafplatz mit der schlafenden Person umfassen,
- Verarbeiten der Tiefenbilder und Ermitteln der Schlafposition durch ein künstliches, neuronales Netzwerk, insbesondere durch ein Convolutional Neural Net, ein Deep Neural Net oder durch ein 3D Convolutional Neural Net und/ oder,
- Ermitteln der Schlafposition, wobei Schlafpositionskenngrößen der Tiefenbilder ermittelt werden und wobei ein trainierter Classifier eines maschinellen Lernens Algorithmus die Schlafposition anhand der bereitgestellten Schlafpositionskenngrößen ermittelt.

Schlafpositionen können beispielsweise in linke Seite, rechte Seite, Bauch, Rücken, und aufrecht klassifiziert werden. Entsprechend ermittelt das erfindungsgemäße Verfahren einen Teil oder alle dieser Schlafpositionen und gibt diese aus.

Das erfindungsgemäße Verfahren ist dabei besonders vorteilhaft, da es die Schlafposition insbesondere berührungsfrei ermittelt und so daher das Schlafverhalten der Person nicht stört.

Gemäß einem Aspekt der Erfindung werden, wenn das Ermitteln der Schlafposition durch einen trainierten Classifier eines maschinellen Lernalgorithmus zum Ermitteln der Schlafposition erfolgt, mindestens die folgenden Schritte durchgeführt:
- Bereichsweise ermitteln von Schlafpositionskenngrößen für jedes Tiefenbild, wobei das jeweilige Tiefenbild dazu in insbesondere gleichförmige und gleichgroße Bereiche unterteilt wird, die das gesamte jeweilige Tiefenbild umfassen, wobei die Schlafpositionskenngrößen für jeden Bereich erfasst werden und wobei die Schlafpositionskenngrößen einen arithmetische und/ oder einen geometrischen Mittelwert, und/ oder Median der Tiefendaten des jeweiligen Bereichs umfassen, und/ oder den höchsten und/ oder den niedrigsten Tiefenwert des jeweiligen Bereichs umfassen, und/ oder einen Mittelwert der n-höchsten Tiefenwerte und/oder m-niedrigsten Tiefenwerte des jeweiligen Bereichs, wobei n und m eine vorbestimmte Zahl, insbesondere 100 ist, und wobei insbesondere für jeden Bereich die Varianz und/ oder die größte principal component der von dem Bereich umfassten Tiefendaten als Schlafpositionskenngröße ermittelt wird.

Erfindungsgemäß kann auch hier eine Vorverarbeitung der Tiefenbilder wie oben beschrieben vorgenommen werden.

Eine beispielhafte Ausführungsform des Verfahrens ist im Folgenden gegeben.

Eine Zeitserie umfasst hier beispielsweise eine Teilmenge an Tiefenbildern aus einer Serie von beispielsweise 900 Tiefenbildern, die mit einer framerate von 30 fps aufgenommen wurden. Aus dieser Serie von 900 Tiefenbildern werden nun insgesamt mindestens 10 solcher Tiefenbilder in gleichem zeitlichen Abstand zu besagter Zeitserie zusammengefasst. Wenn mehr Tiefenbilder, beispielsweise alle Tiefenbilder der Serie von 900 Tiefenbildern zur besagten Zeitserie zusammengefasst werden, dann erhöht dies die benötigte Zeit und den Speicherbedarf für das Training des Classifiers. Das Zeitintervall von 30 Sekunden (900 Frames) ist so gewählt, da die Daten eines Referenz Polysomnographieverfahrens eine solche zeitliche Auflösung haben. Es können auch kürzere Zeitabschnitte gewählt werden, z.B. 15 Sekunden, 10 Sekunden, 5 Sekunden. Dies erhöht allerdings die benötigte Zeit und den Speicherbedarf für das Training des Classifiers, sollte die Anzahl der gesampelten Frames in diesem Zeitintervall nicht angepasst werden.

Über jedes der Tiefenbilder der Zeitserie wird ein gleichmäßiges Raster (Bereiche) gelegt, z.B. mit einer Kantenlänge von 1/10 der Bettlänge, was bei einem Kinect V2 Sensor etwa 40x40 Pixeln entspricht. Für jedes Feld dieses Rasters werden Mittelwert, Median, Maximum, Minimum und Varianz der in diesem Feld enthaltenen Pixelwerte berechnet.

Die so berechneten Werte der einzelnen Rasterfelder, genannt Schlafpositionskenngrößen oder Features, werden zusammen mit vorhandenen Annotationen über die Schlafposition aus einem Referenz Polysomnographie System benutzt, um einen Classifier mit Methoden des maschinellen Lernens zu trainieren. Mögliche Verfahren des maschinellen Lernens und Classifier sind RUSBoost [4] und Entscheidungsbäume [5] oder Subspace [6] K-nearest neighbour classification.

Alternativ können hier auch Deep Neural Networks zur Klassifikation eingesetzt werden. Insbesondere bei steigender Anzahl der Trainingsdaten ist dieser Ansatz sinnvoll, da hier zwar höhere Rechenkapazität für das Training benötigt wird, aber damit auch eine weitere Verbesserung der Klassifikationsergebnisse erzielt werden kann. Konkret kann ein 3D Convolutional Neural Net (3D CNN) eingesetzt werden, welches Tiefenbilder der Zeitserie von z.B. 30 Sekunden oder 900 Frames verarbeitet und daraufhin die darin enthaltene Schlafposition klassifiziert. Ein möglicher Aufbau eines solchen 3D CNN ist beschrieben in [7].

Eine Beispielkonfiguration für ein DNN zur Klassifikation der Schlafposition ist beispielsweise durch das Bereitstellen eines input layers gegeben, welcher beispielsweise Bilder der Größe 25x50 Pixel in Graustufen entgegen nimmt oder entgegen nehmen kann. Damit hat der input layer eine Größe von 1250. Weiterhin können drei hidden layers mit 16, 8 und 4 Neuronen, sowie ein output layer der Größe 4 bereitgestellt werden. Die Verbindungen verlaufen vom input layer zum ersten hidden layer, vom ersten hidden layer zum zweiten hidden layer, vom zweiten zum dritten und von diesem schließlich zum output layer. Es sind jeweils alle Neuronen eines layers mit allen Neuronen des darauffolgenden layers verbunden (sog. feedfoward Architektur). Als Aktivierungsfunktion kann die sog. softmax Funktion verwendet werden. Das Netz kann mit der sog. Glorot Intialisierung [16] initialisiert werden. Als Fehlermaß für das Training kann der mean squared error (MSE) verwendet werden. Die Lernrate kann initial auf 0.1 gesetzt werden, die Abnahme der Lernrate (learning rate decay) auf 0.5.

Alternativ kann auch ein Convolutional Neural Net (CNN) benutzt werden, welches auf, in der Auflösung verringerten, Bildern arbeitet. Dazu werden die oben genannten Mittelwerte oder Mediane des Rasters als Bild ins CNN gegeben. Zum Training des CNN können Bilder, die im zeitlichen Abstand von z.B. 30 Sekunden erfasst wurden, benutzt werden, für die die korrekte Schlafposition aus den einem Polysomnographie Referenzsystem stammt.

Der so trainierte Classifier kann anschließend benutzt werden, um Zeitintervalle mit extrahierten Features von Personen nach Schlafphasen zu klassifizieren. Dafür durchlaufen neue Aufnahmen die gleichen Preprozessing- und Featureextraktionsschritte. Anschließend werden die extrahierten Features mit dem trainierten Classifier klassifiziert. Sollte ein 3D CNN verwendet werden, werden die Videoabschnitte, hier 900 Frames, direkt zur Klassifikation in das 3D CNN gegeben, welches die Klassifikation der Schlafposition für diesen Abschnitt vornimmt.

Das erfindungsgemäße Problem wird weiterhin durch ein Verfahren zur Analyse von Schlafparametern mit einem Tiefensensor gelöst, zumindest umfassend die Schritte:
- Erfindungsgemäße berührungslose Atembewegungsermittlung,
- Erfindungsgemäße berührungslose Beinbewegungsermittlung,
- Erfindungsgemäße berührungslose Schlafphasenermittlung,
- Erfindungsgemäße berührungslose Schlafpositionsermittlung,
wobei die berührungslos ermittelten Bewegungssignale der jeweiligen Körperbereiche, mit der ermittelten Schlafphase und der jeweiligen ermittelten Schlafposition zusammen ausgegeben und/ oder miteinander kombiniert werden, um eine Analyse des Schlafverhaltens der Person zu ermöglichen.

Eine mit diesem Verfahren durchgeführte Analyse von Schlafparametern ist besonders aufgrund der berührungslosen Ermittlung aller Parameter / Signale vorteilhaft gegenüber vergleichbaren nicht-berührungslosen Verfahren, da sie das Schlafverhalten der Person nicht beeinträchtigt.

Insbesondere können durch die Kombination bzw. das gleichzeitige Vorliegen besagter Messdaten / Bewegungssignale oftmals überhaupt erst zuverlässige Aussagen über potenziell pathologisches Schlafverhalten oder krankhaftes Schlafverhalten getroffen werden.

Vorteilhaft kann aus dem Vergleich der ermittelten Bewegungssignale, Schlafphase und Schlafposition nach klinisch etablierten Standards ermittelt werden, ob die Person unter einer Schlafstörung leidet, insbesondere unter einem restless leg movement syndrome (RLS) und/ oder einer obstruktiven oder zentralen Schlafapnoe leidet und wobei insbesondere anhand der Häufigkeit der detektierten pathologischen Bewegungssignale der Beine und des Atems eine Quantifizierung einer Schlafstörung erfolgen kann.

Gemäß einer weiteren Ausführungsform der Erfindung wird das Bewegungssignal aus der Ermittlung der Atembewegung für Intervalle der Zeitserie mit einem Wichtungsfaktor größer 1, insbesondere mit einem Wichtungsfaktor von 2, multipliziert, in denen eine seitliche Schlafposition, insbesondere eine rechts liegende oder links liegende oder eine bauchliegende Schlafposition der Person ermittelt ist.

Durch die Wichtung des Atembewegungssignals wird der insbesondere ein geringerer ermittelter Atembewegungsumfang in den Tiefenbildern korrigiert, so dass ein tatsächlicher Atemumfang ermittelt werden kann, was für die korrekte Auswertung von Atemmustern von großer Bedeutung ist

Vorteilhaft können pathologische Atemstörungen mittels eines Pattern Matching Algorithmus, insbesondere mit dem Wichtungsfaktor gewichteten Bewegungssignal der Atembewegung identifiziert werden. Dieses Atemsignal kann nach unterschiedlichen Störungsarten, insbesondere einer obstruktiven oder zentralen Störung, klassifiziert werden.

Weitere Merkmale und Vorteile der Erfindung werden nachfolgend anhand der Figuren in Zusammenhang mit den oben aufgeführten Ausführungsbeispielen und Ausführungsformen erläutert. Es zeigen:
- Fig. 1: eine schematische Aufstellung des Tiefensensors in Relation zum Schlafplatz der Person mit dem Tiefensensor Koordinatensystem;
- Fig. 2: eine von einem Tiefensensor ermittelte Point-Cloud, die noch nicht auf das Sensorkoordinatensystem normalisiert wurde und somit perspektivische Verzerrungen aufweisen kann.
- Fig. 3: eine Point-Cloud nach Transformation, so dass das der Normalenvektor des Bodens mit der z-Achse des Sensors übereinstimmt.
- Fig. 4: Tiefenbilder (die 3 linken Abbildungen), bei denen jeweils eine Maske (gestrichelte Linie) für den Beinbereich ermittelt wurde. Die Abbildung ganz rechts zeigt eine Aggregationsmaske (durchgezogene Linie), die aus den 3 Masken (gestrichelt) der drei Tiefenbilder zusammengesetzt ist.
- Fig. 5: Bewegungssignal eines Körperbereichs aufweisend Änderungen der Schlafposition und detektierte natürliche Bewegungen (schraffiert hinterlegt)
- Fig. 6: Bewegungssignal des Beinbereichs in Sensorrichtung (links), Bein-Bewegungssignal des Beinbereichs auf der Bettebene (rechts); und
- Fig. 7: Extrahiertes Bewegungssignal des Atembereichs im Vergleich zu einem anderweitig ermittelten Referenzsignal von Apnoe und normaler Atmung.

In Fig. 1 ist schematisch eine Anordnung eines Tiefensensors relativ zu einem Schlafplatz einer Person dargestellt. Die Abbildung zeigt zwei mögliche Positionen des Tiefensensors, die geeignet sind, Atem- und/oder Beinbewegungen, eine Schlafposition und/oder eine Schlafphase mit dem Verfahren zu ermitteln. Dabei sollte der Sensor oberhalb des Schlafplatzes angeordnet sein, und die Person sollte sich im Sichtfeld des Sensors befinden.

In Fig. 1 ist einerseits das Sensorkoordinatensystem zu sehen, welches im Vergleich zu dem Normalenvektor verkippt ist. Daher ist die ursprüngliche Ausgabe einer Point-Cloud aus dem Sensor, so wie es in Fig. 2 dargestellt ist, zunächst perspektivisch verzerrt.

In Fig. 2 ist der Schlafplatz mit einer darauf liegenden Person zu erkennen. Die Darstellung anhand einer Point-Cloud ist dabei das vom Sensor ausgegebene Signal mit den Tiefeninformationen. Zu jedem Punkt der Point-Cloud gibt es einen, dem Punkt zugeordneten Tiefenwert.

Des Weiteren sind in den Randbereichen Teile des Raumes zu sehen, die für die Analyse der Sensordaten unerheblich sind. Dies sind Bereiche, die weder den Schlafplatz noch die Person umfassen.

Um die vom Sensor ermittelte Point-Cloud mit den Tiefeninformationen besser verarbeiten zu können, wird das Sensorkoordinatensystem auf ein Koordinatensystem, das durch den Normalenvektor aufgespannt wird umgerechnet, so dass das resultierende Tiefenbild eine direkte Aufsicht auf den Schlafplatz der Person darstellt. Auf dem Tiefenbild sind der Schlafplatz sowie die Person in seitlicher Schlafposition zu erkennen. Des Weiteren kann ein Bein- und ein Torsobereich identifiziert werden.

In Fig. 4 ist schematisch eine Folge von 3 Bildern einer schlafenden Person dargestellt. Dabei wurde bereits für jedes einzelne Bild eine Maske für die Beine der Person ermittelt (dargestellt durch gestrichelte Linie).

Aus den so ermittelten Masken kann eine Aggregationsmaske ermittelt werden, die alle Masken der Einzelbilder umfasst. Innerhalb dieser Aggregationsmaske finden dann die mess-technische relevanten Änderungen im Tiefenbild statt. Durch die Beschränkung der Auswertung der Daten auf die Aggregationsmaske erhöht sich das Signal zu Rauschverhältnis eines Beinbewegungssignals.

In Fig. 5 ist das Bewegungssignal als Funktion der Zeit dargestellt. Bewegungssignale die auf eine Änderung der Beinposition herrühren, erzeugen vergleichsweise kleine Amplituden im Bewegungssignal, während hingegen eine Änderung der Schlafposition (grau schraffierte Bereiche) ein sehr hohes Bewegungssignal erzeugt. Für die Ermittlung der Beinbewegungen oder Atembewegungen werden die Bereiche aus dem Signal entfernt, die auf Änderungen der Schlafposition zurückzuführen sind. Dies ist in Fig. 6 dargestellt.

Im linken Panel von Fig. 6 ist ein Bewegungssignal des Beinbereichs in Sensorrichtung dargestellt. Im rechten Panel von Fig. 6 ist ein Signal von BeinBewegungen in Richtung der Bettebene dargestellt.

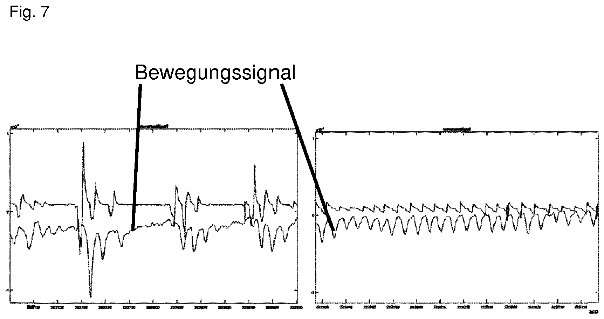

Fig. 7 zeigt ein erfindungsgemäß extrahiertes Bewegungssignal des Atembereichs (untere Linie) im Vergleich zu einem mit einer anderen Methode ermittelten Referenzsignal (obere Linie) von Apnoe (linker Panel) und normaler Atmung (rechter Panel).

Mit der Erfindung können in vorteilhafter Weise Körperbewegungen, wie beispielsweise die Bewegung der Beine oder des Torso, zuverlässig und berührungslos ermittelt werden.

### Referenzliste:

[1] Ridler TW, Calvard S. (1978) Picture thresholding using an iterative selection method, IEEE Trans. System, Man and Cybernetics, SMC-8: 630-632.
[2] Lim, Jae S., Two-Dimensional Signal and Image Processing, Englewood Cliffs, NJ, Prentice Hall, 1990, pp. 469-476.
[3] Krüger, Björn, Anna Vögele, Marouane Lassiri, Lukas Herwartz, Thomas Terkatz, Andreas Weber, Carmen Garcia, Ingo Fietze, and Thomas Penzel. "Sleep Detection Using De-Identified Depth Data." Journal of Mobile Multimedia 10, no. 3&4 (December 2014): 327-42.
[4] Seiffert, C., T. Khoshgoftaar, J. Hulse, and A. Napolitano. RUSBoost: Improving clasification performance when training data is skewed. 19th International Conference on Pattern Recognition, pp. 1-4, 2008.
[5] Breiman, L., J. Friedman, R. Olshen, and C. Stone. Classification and Regression Trees. Boca Raton, FL: CRC Press, 1984.
[6] Ho, T. K. The random subspace method for constructing decision forests. IEEE Transactions on Pattern Analysis and Machine Intelligence, Vol. 20, No. 8, pp. 832-844, 1998.
[7] 3D Convolutional Neural Networks for Human Action Recognition, Shuiwang Ji, Wei Xu, Ming Yang, Kai Yu
[8] Shotton, Jamie, Toby Sharp, Alex Kipman, Andrew Fitzgibbon, Mark Finocchio, Andrew Blake, Mat Cook, and Richard Moore. "Real-Time Human Pose Recognition in Parts from Single Depth Images." Commun. ACM 56, no. 1 (January 2013): 116-124. doi:10.1145/2398356.2398381.
[9] Felzenszwalb, Pedro F., Ross B. Girshick, David McAllester, and Deva Ramanan. "Object Detection with Discriminatively Trained Part-Based Models." Pattern Analysis and Machine Intelligence, IEEE Transactions on 32, no. 9 (2010): 1627-1645.
[10] Sepp Hochreiter, Jürgen Schmidhuber (1997). "Long short-term memory". Neural Computation. 9 (8): 1735-1780. doi:10.1162/neco.1997.9.8.1735.
[11] Marvin Minsky, Seymour Papert: Perceptrons. An Introduction to Computational Geometry. MIT Press, Cambridge MA u. a. 1969.
[12] D. E. Rumelhart, G. E. Hinton, and R. J. Williams, "Learning representations by back-propagating errors," Nature vol. 323, pp. 533-536, 1986.
[13] Deng, L.; Yu, D. (2014). "Deep Learning: Methods and Applications" (PDF). Foundations and Trends in Signal Processing. 7 (3-4): 1-199. doi:10.1561/2000000039
[14] LeCun, Y., Bottou, L., Bengio, Y., and Haffner, P. (1998d). Gradient-based learning applied to document recognition. Proceedings of the IEEE, 86(11), 2278-2324.
[15] Sepp Hochreiter; Jürgen Schmidhuber (1997). "Long short-term memory" (PDF). Neural Computation. 9 (8): 1735-1780. doi:10.1162/neco.1997.9.8.1735. PMID 9377276.
[16] Xavier Glorot & Yoshua Bengio's Understanding the difficulty of training deep feedforward neural networks. 2010
[17] Diederik Kingma, Jimmy Ba Adam: A Method for Stochastic Optimization , CoRR 2014

## Patentansprüche

1. Verfahren zur berührungslosen Ermittlung von Atem- und Beinbewegungen schlafender Personen mit einem Tiefensensor, umfassend die Schritte:
- Bereitstellen einer insbesondere vorverarbeiteten Zeitserie von Tiefenbildern des Tiefensensors, die einen Schlafplatz mit der schlafenden Person umfassen,
- Ermitteln und Quantifizieren zumindest eines Bewegungssignals, das aus Bewegungen mindestens eines Körperbereichs der schlafenden Person aus der Zeitserie der Tiefenbilder ermittelt wird, wobei der mindestens eine Körperbereich den Torso, insbesondere den Brustbereich der Person umfasst und/ oder wobei der mindestens eine Körperbereich den Beinbereich der Person umfasst.

2. Verfahren gemäß Anspruch 1, **dadurch gekennzeichnet, dass** zum Ermitteln des mindestens einen Bewegungssignals des mindestens einen Körperbereichs der Person in jedem Tiefenbild der Zeitserie eine Maske umfassend den jeweiligen Körperbereich ermittelt wird und diesem Körperbereich zugeordnet wird.

3. Verfahren gemäß Anspruch 2, **dadurch gekennzeichnet, dass** die zugeordneten Masken zeitbereichsweise zu einer Serie von Aggregationsmasken zusammengefasst werden, wobei die jeweilige Aggregationsmaske den jeweiligen Tiefenbildern des jeweiligen Zeitbereichs zugeordnet wird.

4. Verfahren gemäß Anspruch 3, **dadurch gekennzeichnet, dass** für jedes Tiefenbild aus einem Bildbereich, der innerhalb der jeweiligen zugeordneten Aggregationsmaske liegt, mindestens eine Bewegungskenngröße für den mindestens einen Körperbereich ermittelt wird und dem jeweiligen Tiefenbild zugeordnet wird.

5. Verfahren gemäß Anspruch 4, **dadurch gekennzeichnet, dass** die mindestens eine Bewegungskenngröße, wenn das mindestens eine Bewegungssignal aus den Atembewegungen ermittelt wird, ein Mittelwert der Tiefendaten über dem von der Aggregationsmaske umfassten Bildbereich des jeweiligen Tiefenbildes ist, wobei das zur Atembewegung zugehörige mindestens eine Bewegungssignal die zeitliche Folge der Bewegungskenngröße umfasst.

6. Verfahren gemäß Anspruch 4 oder 5, **dadurch gekennzeichnet, dass** das zur Atembewegung zugehörige mindestens eine Bewegungssignal mit Atembewegungs-Referenzmustern verglichen wird und so insbesondere auf pathologische Merkmale untersucht werden kann oder dass das zur Atembewegung zugehörige mindestens eine Bewegungssignal die Amplitude mit Hilfe von Hüllkurven ermittelt wird, wobei insbesondere diese Amplitude mit Hilfe von Schwellwerten auf pathologische Merkmale untersucht wird.

7. Verfahren gemäß Anspruch 4, **dadurch gekennzeichnet, dass** die mindestens eine Bewegungskenngröße, wenn das mindestens eine Bewegungssignal aus den Beinbewegungen ermittelt wird, ein Mittelwert der Tiefendaten über dem von der Aggregationsmaske umfassten Bildbereich des jeweiligen Tiefenbildes ist und/ oder wobei die mindestens eine Bewegungskenngröße eine Größe, eine Position und/ oder eine Bewegungsrichtung der dem jeweiligen Tiefenbild zugeordneten Maske ist, und/ oder wobei die mindestens eine Bewegungskenngröße eine Position und/ oder eine Bewegungsrichtung von Merkmalen innerhalb der jeweiligen Maske ist, wobei die Position und/ oder Bewegungsrichtung besagter Merkmale insbesondere durch Verwendung eines optical flow Algorithmus ermittelt wird, wobei das zur Beinbewegung zugehörige mindestens eine Bewegungssignal die zeitliche Folge der mindestens einen Bewegungskenngröße umfasst und wenn mehrere Bewegungskenngrößen ermittelt werden, jede zeitliche Folge einer solchen Bewegungskenngröße einem Bewegungssignal zugeordnet wird.

8. Verfahren nach Anspruch 7, **dadurch gekennzeichnet, dass** für jedes zur Beinbewegung zugehörige mindestens eine Bewegungssignal Spitzen mit einem Spitzendetektionsverfahren ermittelt werden, und wobei insbesondere anhand der Verteilung und der Höhe der Spitzen in dem jeweiligen mindestens einen Bewegungssignal Bewegungen der Beine detektiert und klassifiziert werden, insbesondere wird der Periodic Limb Movement Index Score (PLMI) als durchschnittliche Anzahl der Periodic Limb Movements pro Stunde ermittelt.

9. Verfahren nach einem der Ansprüche 2 bis 8, **dadurch gekennzeichnet, dass** die Ermittlung der Maske für den mindestens einen Körperbereichs mit einem, insbesondere überwachten, maschinellen Lernalgorithmus, insbesondere einem Segmentationsalgorithmus erfolgt.

10. Verfahren zur berührungslosen Klassifikation von Schlafphasen einer schlafenden Person mit einem Tiefensensor, aufweisend zumindest die Schritte:
- Bereitstellen einer insbesondere vorverarbeiteten Zeitserie von Tiefenbildern umfassend Tiefendaten des Tiefensensors, die einen Schlafplatz mit der schlafenden Person umfassen,
- Verarbeiten der Tiefenbilder und Ermitteln der Schlafphase durch ein künstliches, neuronales Netzwerk, insbesondere durch ein 3D Convolutional Neural Net oder ein Long short-term memory Neural Net, und/oder
- Ermitteln der Schlafphase, wobei Schlafphasenkenngrößen der Tiefenbilder ermittelt werden und wobei ein trainierter Classifier eines maschinellen Lernens Algorithmus die Schlafphase anhand der bereitgestellten Schlafphasenkenngrößen ermittelt.

11. Verfahren zur berührungslosen Klassifikation einer Schlafposition einer schlafenden Person mit einem Tiefensensor, aufweisend die Schritte:
- Zur Verfügung stellen einer insbesondere vorverarbeiteten Zeitserie von Tiefenbildern des Tiefensensors, die einen Schlafplatz mit der schlafenden Person umfassen,
- Verarbeiten der Tiefenbilder und Ermitteln der Schlafposition durch ein künstliches, neuronales Netzwerk, insbesondere durch ein 3D Convolutional Neural Net, ein Convolutional Neural Net oder ein Long short-term memory Neural Net und/ oder,
- Ermitteln der Schlafposition, wobei Schlafpositionskenngrößen der Tiefenbilder ermittelt werden und wobei ein trainierter Classifier eines maschinellen Lernens Algorithmus die Schlafposition anhand der bereitgestellten Schlafpositionskenngrößen ermittelt.

12. Verfahren zur Analyse von Schlafparametern mit einem Tiefensensor, umfassend die Schritte:
- Berührungslose Atembewegungsermittlung, nach einem der Ansprüche 1 bis 9,
- Berührungslose Beinbewegungsermittlung, nach einem der Ansprüche 1 bis 9,
- Berührungslose Schlafphasenermittlung, gemäß Anspruch 10,
- Berührungslose Schlafpositionsermittlung, gemäß Anspruch 11,
wobei die berührungslos ermittelten Bewegungssignale der jeweiligen Körperbereiche, mit der ermittelten Schlafphase und der jeweiligen ermittelten Schlafposition zusammen ausgegeben und/ oder miteinander kombiniert werden, um eine Analyse des Schlafverhaltens der Person zu ermöglichen.

13. Verfahren gemäß Anspruch 12, **dadurch gekennzeichnet, dass** das Bewegungssignal aus der Ermittlung der Atembewegung für Intervalle der Zeitserie mit einem Wichtungsfaktor größer 1, insbesondere mit einem Wichtungsfaktor von 2, gewichtet, insbesondere multipliziert wird, in denen eine seitliche oder bauchliegende Schlafposition der Person ermittelt wurde.
